(19) Europäisches Patentamt
European Patent Office
Office européen des brevets

(11) Veröffentlichungsnummer: **0 228 675 B1**

(12) # EUROPÄISCHE PATENTSCHRIFT

(45) Veröffentlichungstag der Patentschrift: **16.05.90**

(51) Int. Cl.[5]: **C07C 45/58**, C07C 47/24, C07C 323/01, C07C 45/65, C07C 45/52, C07C 47/277

(21) Anmeldenummer: **86117725.1**

(22) Anmeldetag: **19.12.86**

(54) **Neue Phenylacetaldehyde und Verfahren zur Herstellung von Phenylacetaldehyden.**

(30) Priorität: **31.12.85 DE 3546372**

(43) Veröffentlichungstag der Anmeldung: **15.07.87 Patentblatt 87/29**

(45) Bekanntmachung des Hinweises auf die Patenterteilung: **16.05.90 Patentblatt 90/20**

(84) Benannte Vertragsstaaten: **AT BE CH DE FR GB IT LI LU NL SE**

(56) Entgegenhaltungen:
**EP-A- 0 100 117**

**CHEMICAL AND PHARMACEUTICAL BULLETIN, Band 18, Nr. 10, 1970, Seiten 2028-2037, Tokyo, JP; H. KUGITA et al.: "Synthesis of 1,5-Benzothiazepine derivatives. I"**
**PATENT ABSTRACTS OF JAPAN, Band 10, Nr. 350 (C-387)[2406], 26. November 1986; & JP - A - 61 151 145**

(73) Patentinhaber: **BASF Aktiengesellschaft, Carl-Bosch-Strasse 38, D-6700 Ludwigshafen(DE)**

(72) Erfinder: **Hoelderich, Wolfgang, Dr., Mannheimer Strasse 18 c, D-6710 Frankenthal(DE)**
Erfinder: **Goetz, Norbert, Dr., Schoefferstrasse 25, D-6520 Worms 1(DE)**
Erfinder: **Hupfer, Leopold, Dr., Waltershöhe 3, D-6701 Friedelsheim(DE)**
Erfinder: **Kropp, Rudolf, Sprottauer Strasse 2, D-6703 Limburgerhof(DE)**
Erfinder: **Theobald, Hans, Dr., Queichstrasse 6, D-6703 Limburgerhof(DE)**
Erfinder: **Wolf, Bernd, Dr., Eichenstrasse 11, D-6704 Mutterstadt(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue substituierte Phenylacetaldehyde, die als wertvolle Zwischenprodukte zur Herstellung neuer Wirkstoffe in Insektiziden dienen. Weiterhin betrifft die Anmeldung ein Verfahren zur Herstellung dieser Phenylacetaldehyde.

Bekannte Verfahren zur Herstellung von Phenylacetaldehyden, die auch im technischen Maßstabe ausgeübt werden können, sind:

a) Die Dehydrierung von Phenylethanolen: Nur Teilumsatz ist möglich, verlustreiche Trennung von Ausgangs- und Endprodukt (Phenylacetaldehyde sind thermolabil), bei Fraktionierung Bildung von Selbstkondensationsprodukten.
Die Herstellung von halogenhaltigen Phenylacetaldehyden auf diesem Wege ist nicht möglich, da unter den Reaktionsbedingungen Halogenabspaltung erfolgt.

b) Die Umlagerung von Styroloxiden: In der Regel ebenfalls nur Teilumsatz, schwer abtrennbare Nebenprodukte, schlechte Selektivitäten, schlechte Standzeiten der bisher benutzten Katalysatoren aufgrund von Oberflächenbelegungen.

In EP 100 117 wird die Reaktion von Styroloxid und von am aromatischen Kern alkyl- bzw. alkoxylsubstituierte Styroloxiden an einem Titan-haltigen Zeolithen zu β-Phenylaldehyden in der flüssigen Phase bei 30 bis 100°C beschrieben. Der hierzu eingesetzte Katalysator muß aufwendig aus teuren hochreinen Edukten wie Tetraalkylorthosilikat, Tetralkylorthotitanat und Tetrapropylammoniumhydroxid hergestellt werden. Hoher Umsatz wird nur erzielt, wenn die Reaktion in einem Lösungsmittel wie Methanol oder Aceton bei 30 bis 100°C in der Flüssigphase stattfindet und Verweilzeiten von 1 bis 1,5 h eingehalten werden. Dies zieht erhöhte Destillations- und Betriebskosten nach sich. Weiterhin besitzt die Reaktion an Titan-haltigen Zeolithen keine Allgemeingültigkeit und ist nur bei Styroloxid und am Aromaten alkylierten bzw. alkoxylierten Styroloxiden möglichen.

Andere Arbeiten zur Umlagerung von Epoxiden zu Carbonylverbindungen sind bekannt. Beispielsweise Cyclododecanon wird an Pd-oder Rd-dot. $Al_2O_3$ aus Epoxycyclododecan erhalten. In dieser Arbeit wird ausdrücklich darauf hingewiesen, daß Zeolithe für diese Reaktion ungeeignet sind. Auch der Einsatz von A-Zeolithen für die Umlagerung von Butylenoxid zu Butyraldehyd (55 bis 72 %) ist beschrieben. Die Selektivität läßt noch zu wünschen übrig. Auch läßt sich der A-Zeolith-Katalysator nach seiner Desaktivierung durch Koks schwer regenerieren, da bei den hierfür notwendigen Temperaturen von ca. 500°C die Kristallstruktur dieses Zeolithen zerstört wird. Weiterhin ist es für die Umsetzung von Propylenoxid zu Aceton oder Propionaldehyd an alkali-dotierten X-Zeolithen notwendig, in Abwesenheit stark acider Zentren zu arbeiten.

Auch ist bekannt, daß man Phenylacetaldehyde durch Umlagerung von Styrolglykol an Alumniniumsilikaten mit $SiO_2$:$Al_2O_3$ = 80:20 - 93:7 gemischt mit z.B. Eisen-, Calcium- bzw. Magnesiumoxid oder an aktiviertem Ton in Suspension unter vermindertem Druck erhalten kann. Diesen beiden Verfahren ist gemeinsam, daß die Ausbeuten mit 50 bis 86% noch verbesserungswürdig sind. Weiterhin wird über die Standzeit und Regenerierbarkeit der Katalysatoren keine Aussage gemacht. Auch ist dieses Verfahren nicht flexibel; denn halogenierte Verbindungen werden nicht erhalten. Im Falle des Tons handelt es sich um ein natürliches Mineral, das je nach Provenience unterschiedliche Zusammensetzung und damit unterschiedliche katalytische Eigenschaften und Selektivität aufweist. Dies erschwert insbesondere ein kontinuierliches technisches Verfahren.

Aldehyde lassen sich auch in einer Rosenmund-Reduktion aus Carbonsäurechloriden gewinnen. Derartige Reaktionen gehen gut in der Flüssigphase mit Arylsäurechloriden. Bei anderen Säurechloriden wie z.B. Aralkylcarbonsäurechloriden sind in der Regel niedrigere Ausbeuten gepaart mit Katalysatorvergiftung anzutreffen.

Die Erfindung betrifft Phenylacetaldehyde der Struktur(I)

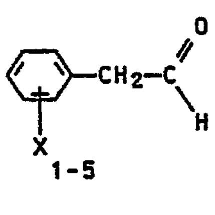

(I)

wobei die Substituenten X Difluor-, Trifluor-, Tetrafluor-, p-Trifluormethyl-, 2-Methyl-4-fluor-, Halogenalkoxy- oder Halogenalkylthio-Reste oder nebeneinander vorhandener Cl-, F-, $CF_3$-, Alkyl-, Alkoxy-, Halogenalkoxy- und/oder Halogenalkylthio-Reste bedeuten.

Die vorliegende Erfindung betrifft weiterhin Verfahren zur Herstellung der Phenylacetaldehyde der Struktur(I) aus gut zugänglichen Ausgangsstoffen in Gegenwart von Katalysatoren, die sich durch einfache Verfügbarkeit, hohe Aktivität und leichte Regenerierbarkeit auszeichnen. Weiterhin sind bei langen Katalysatorstandzeiten hohe Umsätze, hohe Selektivitäten und eine flexible Einsetzbarkeit des Katalysators hinsichtlich der Edukte gewährleistet.

Im erfindungsgemäßen Verfahren werden die eingangs erwähnten Nachteile der bisherigen Verfahrensweisen ausgeräumt und die an die Katalysatoren gestellten Anforderungen erfüllt. Im Hinblick auf den Stand der Technik ist das Gelingen des Verfahrens umso überraschender, da man bisher nur schwach acide X-Zeolithe einsetzte bzw. Zeolithe für Umlagerungsreaktionen als ungeeignet einstufte. Daher konnte man nicht erwarten, daß gerade mit Zeolithen, die sich durch hohe Acidität sowie durch strenge Strukturparameter auszeichnen, in so weiten Grenzen und bei so großer Vielfalt der Edukte solch hervorragende Ergebnisse erhalten werden.

Vorteile des erfindungsgemäßen Verfahrens bei der Umlagerung an den erfindungsgemäßen Katalysatoren sind: Vollständiger Umsatz, keine Trennprobleme, lange Standzeiten, Selektivitäten > 90 %, auch sehr gute Ausbeuten bei halogen-haltigen Ausgangsprodukten, einfache Isolierung der Endprodukte, in der Regel Weiterverwendung ohne zusätzliche Reinigung, leichte Regenerierbarkeit der Katalysatoren bei eventuell auftretender Verkokung. Die Rosenmund Reduktionn war bisher nur bei der Hydrierung von aromatischen Carbonsäurechloriden zu Benzaldehyden von Bedeutung; es ist überraschend, daß auch empfindliche halogenhaltige Phenylacetaldehyde sich nach dieser Methode herstellen lassen; die direkte Weiterverarbeitung der erhaltenen Rohprodukte ist möglich; die Ausgangsstoffe sind gut zugänglich; eine Katalysatorvergiftung ist bei den erfindungsgemäßen Katalysatoren nicht festzustellen.

Die erfindungsgemäßen Verfahren sind:

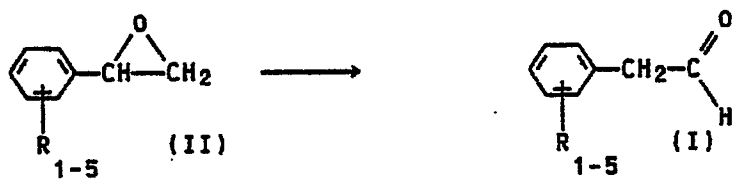

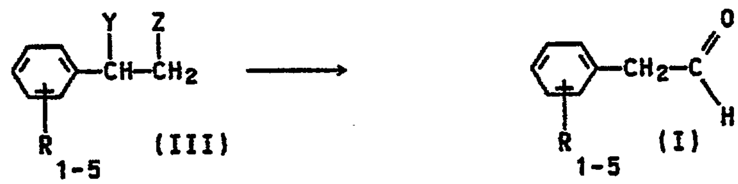

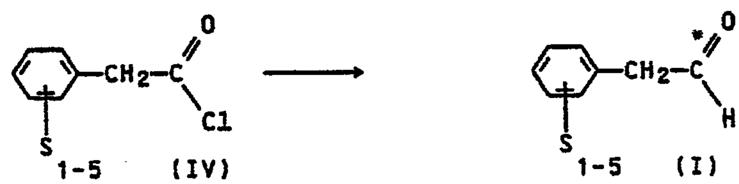

R kann Wasserstoff-, Alkyl-, Alkoxy-, Halogen-, Halogenalkyl-, Halogenalkoxy- und/oder Halogenalkylthio-Reste bedeuten;
Y und Z können untereinander gleich oder verschieden sein und Hydroxy-, Alkoxy-, Aryloxy-, Acyloxy-Reste bedeuten;
S kann Halogen-, Halogenalkyl-, Halogenalkoxy- und/oder Halogenalkylthio-Reste bedeuten.

Die für das erfindungsgemäße Verfahren eingesetzten Epoxide sind z.B. Styroloxid, p-Fluor-styroloxid, p-Chlor-styroloxid, 2,4-Difluor-styroloxid, 3,4-Difluor-styroloxid, 2,4-Dichlor-styroloxid, 2,4,5-Trifluorstyroloxid, o-, m-, p-Trifluormethyl-styroloxid, o-, m, p-Methyl-styroloxid, o-, m-, p-Methoxystyroloxid, 2,3,4,5-Tetrafluor-styroloxid, p-Trifluormethoxy-styroloxid, p-Trifluormethylthio-styroloxid, 2-Fluor-6-chlorstyroloxid, 2-Fluor-4-trifluormethylstyroloxid, 2-Fluor-4-trifluormethoxystyroloxid und 2-Methyl-4-fluor-styroloxid.

Die für das erfindungsgemäße Verfahren eingesetzten Phenylglykole sind z.B. Phenylglykol, Phenylglykolmonomethylether, Phenylglykolessigsäureester und Phenylglykolmonophenylether.

Die für das erfindungsgemäße Verfahren eingesetzten aromatischen Carbonsäurechloride sind z.B. Phenylessigsäurechlorid, o-, m-, p-Phenylacetylchlorid, o-, m-, p-Fluorphenylacetylchlorid, 2-Chlor-6-fluor-phenylacetylchlorid und o-, m-, p-Trifluormethyl-phenyl-acetylchlorid.

Die oben genannten Verbindungen stellen eine Auswahl verwendbarer Komponenten zur Herstellung von substituierten Phenylacetaldehyden dar und sollen die Anwendungsbreite des erfindungsgemäßen Verfahrens für eine Vielzahl von Phenylacetaldehyden nicht einschränken.

Als Katalysatoren für die erfindungsgemäße Umwandlung von Epoxiden werden acide zeolithische Katalysatoren eingesetzt. Zeolithe sind kristalline Alumninosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2 (s. Ullmanns Encyklopädie der techn. Chemie, 4. Auflage, Band 24, S. 575 (1983)). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekeln besetzt.

In den Zeolithen können anstelle von Aluminium auch andere Elemente wie B, Ga, Fe, Cr, Be, As, Sb in das Gitter eingebaut werden oder das Silicium kann durch ein vierwertiges Element wie Ge ersetzt werden.

Als Katalysatoren kommen Zeolithe aus der Mordenit-Gruppe oder engporige Zeolithe vom Erionit- bzw. Chabasit-Typ in Betracht. Besonders vorteilhaft für das erfindungsgemäße Verfahren sind Zeolithe vom Pentasil-Typ. Diese Zeolithe können unterschiedliche chemische Zusammensetzung aufweisen. Es handelt sich hierbei um Alumino-, Boro-, Eisen-, Gallium-, Chrom-, Berylium-, Arsen-, Antimon- und Wismutsilikatzeolithe oder deren Gemische sowie um Alumino-, Boro-, Gallium- und Eisengermanatzeolithe oder deren Gemische.

Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung und Polyaminlösung, insbesondere in 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Auch gehören hierzu die isotaktischen Zeolithe nach DE-OS 3 006 471. Die erhaltenen Aluminosilikatzeolithe enthalten je nach Wahl der Einsatzstoffmengen ein $SiO_2/Al_2O_3$-Verhältnis von 10 bis 40 000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200°C unter autogenem Druck synthetisiert, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit oder insbesondere ohne Alkali- oder Erdalkalizusatz zur Reaktion bringt. Auch gehören hierzu die isotaktischen Zeolithe nach DE-OS 3 006 471. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220°C unter autogenem Druck.

Die so hergestellten Alumino-, Boro- und Eisensilikatzeolithe können nach ihrer Isolierung, Trocknung bei 100 bis 160°C, vorzugsweise 110°C und Calcinierung bei 450 bis 550°C, vorzugsweise 500°C bis 540°C, mit einem Bindemittel im Verhältnis 90:10 bis 40:60 Gew. % zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 95:5, bevorzugt 75:25, Siliciumdioxid, bevorzugt hochdisperses $SiO_2$, Gemische aus hochdispersem $SiO_2$ und hochdispersem $Al_2O_3$ sowie Ton. Nach der Verformung werden die Extrudate oder Preßlinge bei 110°C/116 h getrocknet und bei 500°C/16 h calciniert.

Man erhält auch vorteilhaft Katalysatoren, wenn der isolierte Alumino- bzw. Borosilikatzeolith direkt nach der Trocknung verformt wird und erstmals nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithische Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550°C, bevorzugt 500°C bis 540°C, zu regenerieren. Die Zeolithe erhalten dadurch ihre Anfangsaktivität zurück. Durch partielle Verkokung (pre-coke) ist es möglich, die Aktivität des Katalysators für ein Selektivitätsoptimum des gewünschten Reaktionsproduktes einzustellen.

Um eine möglichst große Selektivität, hohen Umsatz sowie lange Standzeiten zu erreichen, ist es mitunter vorteilhaft, die Zeolithe zu modifizieren. Eine geeignete Modifizierung der Katalysatoren besteht

z.B. darin, daß man den unverformten oder verformten Zeolithen mit Metallsalzen durch einen Ionenaustausch oder durch Imprägnierung dotiert.

Zweckmäßigerweise führt man die Dotierung so durch, daß man z.B. den verformten Pentasilzeolithen in einem Steigrohr vorlegt und bei 20 bis 100°C z.B. eine wäßrige oder ammoniakalische Lösung eines Halogenids oder eines Nitrats der Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cs_2O_3$ in Wasser löst. Mit dieser Lösung wird der verformte oder unverformte Zeolith eine gewisse Zeit, ca. 30. Minuten, getränkt. Die eventuell überstehende Lösung wird am Rotationsverdampfer von Wasser befreit. Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z.B. eine ammoniakalische $Pd(NO_3)_2$-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcinierung bei ca. 500°C kann das so gewonnene zeolithische Material mit oder ohne Bindemittel zu Strängen, Pellets oder Wirbelgut weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z.B. eine ammoniakalische $Pd(NO_3)_2$-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert.

Bei manchen metalldotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Weitere Katalysatoren für die Herstellung von Phenylacetaldehyden aus entsprechenden Epoxiden bzw. Glykolen sind im folgenden beschrieben:

Als Aluminumphosphat-Katalysatoren werden für das erfindungsgemäße Verfahren insbesondere unter hydrothermalen Bedingungen synthetisierte Aluminiumphosphate eingesetzt.

Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP 132 708, US 4 310 440 und US 4 473 663 beschrieben.

Beispielsweise das $AlPO_4$-5 (APO-5) wird synthetisiert, indem man Orthophosphorsäure mit Pseudoboehmit (Catapal SB®) in Wasser homogen mischt; zu dieser Mischung Tetrapropylammoniumhydroxid gibt und danach bei ca. 150°C 20 bis 60 h unter autogenem Druck in einem Autoklaven umsetzt. Das abfiltrierte $AlPO_4$ wird getrocknet bei 100 bis 160°C und calciniert bei 450 bis 550°C.

$AlPO_4$-9 (APO-9) wird ebenfalls aus Orthophosphorsäure und Pseudoboehmit aber in wäßriger DABCO-Lösung (1,4-Diazabicyclo-(2,2,2)octan) bei ca. 200°C unter autogenem Druck während 200 bis 400 h synthetisiert.

Die Synthese des $AlPO_4$-21 (APO-21) erfolgt aus Orthophosphorsäure und Pseudoboehmit in wäßriger Pyrrolidon-Lösung bei 150 bis 200°C unter autogenem Druck während 50 bis 200 h.

Die für das erfindungsgemäße Verfahren eingesetzten Siliciumaluminiumphosphate sind z.B. SAPO-5, SAPO-11, SAPO-31 und SAPO-34. Die Synthese dieser Verbindung wird z.B. in EP 103 117, US 4 440 871 beschrieben. SAPO's werden hergestellt durch Kristallisation aus wäßriger Mischung bei 100 bis 250°C und autogenem Druck während 2 h bis 2 Wochen, wobei die Reaktionsmischung aus einer Silicium, Aluminium- und Phosphorkomponente in wäßrigen aminoorganischen Lösungen umgesetzt wird.

SAPO-5 beispielsweise wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200°C während 20 bis 200 h unter autogenem Druck in einem Rührautoklaven erhalten. Die Trocknung des abfiltrierten Pulvers erfolgt bei 110 bis 160°C und die Calcination bei 450 bis 550°C.

Als Siliciumaluminiumphosphate sind z.B. ZYT-5, ZYT-6, ZYT-7, ZYT-9, ZYT1-11 und ZYT-12 geeignet (J 5 9217-619).

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- oder Dampfatmosphäre bei 250 bis 650°C vorzugsweise 300 bis 500°C herstellen.

Phosphorsäure oder Borsäure wird auf $SiO_2$-, $Al_2O_3$- oder Bims-Träger z.B. durch Auftränken oder Versprühen aufgebracht. Ein phosphorsäure-haltiger Katalysator kann beispielsweise durch Auftränken von $H_3PO_4$- oder $NaH_2PO_4$- oder $Na_2HPO_4$-Lösung auf $SiO_2$ und anschließende Trocknung bzw. Calcination erhalten werden. Phosphorsäure kann aber auch mit Kieselgel zusammen in einem Sprühturm versprüht werden, danach schließt sich eine Trocknung und meist eine Calcination an. Phosphorsäure kann auch in einer Imprägniermühle auf das Trägermaterial aufgesprüht werden.

Die hier beschriebenen Katalysatoren können wahlweise als 2 bis 4 mm Stränge oder als Tabletten mit 3 bis 5 mm Durchmesser oder als Pulver mit Teilchengrößen von 0,1 bis 0,5 mm oder als Wirbelkontakt eingesetzt werden.

Für die erfindungsgemäß angewandte Rosenmund-Reduktion eignen sich Palladiumkatalysatoren, wobei das Palladium auf unterschiedlichen Trägern wie z.B. Aktivkohle, Zeolithe, Siliciumdioxide, Aluminiumoxide, Titanoxide, Zirkondioxide aufgebracht ist, und Zusätze von tert. Aminbasen wie tert. Butylamin, Pyridin, Chinolin und Chalkogenide wie Schwefel, Selen und Tellur zugegeben werden.

Die für die erfindungsgemäße Umwandlung der Epoxide bzw. Glykole in der Regel gewählten Reaktionsbedingungen sind in der bevorzugten Gasphase 200 bis 500°C, vorzugsweise 200 bis 400°C, und eine Belastung WHSV = 0,1 bis 20 $h^{-1}$, bevorzugt 0,5 bis 5 $h^{-1}$ (g Epoxide/g Katalysator und Stunde). Im allgemeinen steigt der Umsatz mit steigender Temperatur stark an, während die Selektivität in einem bestimmten Temperaturbereich nur wenig zurückgeht.

Auch ist es möglich, die Reaktion in der Flüssigphase (Supension-, Riesel- oder Sumpffahrweise) durchzuführen.

Das Verfahren wird in der Regel bei Normaldruck oder je nach Flüchtigkeit der Ausgangsverbindung bei vermindertem oder erhöhtem Druck durchgeführt, wobei die Durchführung vorzugsweise kontinuierlich erfolgt.

Schwerflüchtige oder feste Edukte werden in gelöster Form z.B. in THF-, Toluol- oder Petrolether-Lösung zum Einsatz gebracht. Generell ist auch eine Verdünnung mit Lösungsmitteln wie z.B. voranstehend genannt möglich.

Die Rosenmund-Reduktion wird in der Flüssigphase bei 0 bis 130°C, bevorzugt bei 25 bis 100°C, durchgeführt. Die Reaktion wird meist in Lösung durchgeführt, wobei als Lösungsmittel Kohlenwasserstoffe, bevorzugt Toluol, Xylol, Benzol, Ethylbenzol und Diethylbenzol, verwendet werden. Die Verfahrensweise bei der Rosenmund-Reduktion ist z.B. folgende:

Das Carbonsäurechlorid wird gelöst in einem inerten Lösungsmittel, beispielsweise Xylol, und in Gegenwart eines Palladium-Katalysators hydriert. Der abgespaltete Chlorwasserstoff entweicht gasförmig aus dem Reaktionsgemisch oder wird im Falle der Anwesenheit von Natriumacetat oder einer tertiären organischen Base gebunden. Nach Filtration des Katalysators und gegebenenfalls Natriumchlorid beziehungsweise dem Hydrochlorid der tertiären organischen Base wird der Aldehyd durch Destillation des Filtrats gewonnen.

Man kann aber auch das Filtrat direkt, also ohne Isolierung des Aldehyds, in eine Folgereaktion beispielsweise Acetalisierung einsetzen.

Nach der Umsetzung werden die entstandenen Phenylacetaldehyde nach üblichen Techniken, z.B. durch Destillation aus dem Reaktionsgemisch isoliert; nichtumgesetzte Edukte werden gegebenenfalls, wenn nötig, für die erfindungsgemäße Umsetzung zurückgeführt. Eine direkte Weiterverarbeitung der Reaktionsprodukte ist aufgrund der sehr hohen Ausbeuten möglich. Bei dem erfindungsgemäßen Verfahren fallen bevorzugt die monomeren Verbindungen an. Sollten jedoch auch Oligomere z.B. trimere Phenylacetaldehyde gebildet werden, so können diese abgetrennt und zu den gewünschten Monomeren nach bekannten Methoden gespalten werden.

Die nach dem erfindungsgemäßen Verfahren zugänglichen Verbindungen sind wichtige Zwischenprodukte für biologisch aktive Verbindungen, z.B. Insektizide wie Resmethrin. Weiterhin lassen sie sich z.B. leicht zu Aminen, Alkoholen und Säuren nach dem Fachmann geläufigen Methoden, z.B. durch Oxidation mit Sauerstoff oder durch Reduktion z.B. durch katalytische Hydrierung oder aminierende Hydrierung weiterverarbeiten, die ihrerseits wertvolle Zwischenprodukte darstellen.

Bislang unbekannte Verbindungen lassen sich nach dem erfindungsgemäßen Verfahren herstellen. Diese Verbindungen sind mit ihren physikalischen bzw. spektroskopischen Kenndaten in Tabelle 6 zusammengestellt. Die bereits bekannten Verbindungen ihrerseits sind in Tabelle 7 aufgeführt.

Die Herstellung der Epoxide kann entweder durch Epoxidation der entsprechenden Styrole oder aus Halogenacetophenonen durch Hydrieren zu Chlor- oder Bromhydrinen und durch anschließenden Ringschluß im alkalischen Medium erfolgen. Durch Umsetzung der Epoxide mit Wasser, Alkoholen, Carbonsäuren oder Phenolen lassen sich weitere Vorprodukte herstellen, die sich zu Phenylacetaldehyden umlagern lassen.

Die folgenden Beispiele veranschaulichen die Erfindung.

<u>Beispiele 1 bis 33</u>

Die Reaktionen werden unter isothermen Bedingungen in einem Rohrreaktor (Wendel 0,6 cm Durchmesser, 90 cm Länge) in der Gasphase mindestens 6 Stunden lang durchgeführt. Die Trennung und Charakterisierung der Reaktionsprodukte erfolgt nach üblichen Methoden. Die quantitative Bestimmung der Reaktionsprodukte und der Ausgangsstoffe erfolgte gaschromatographisch und durch CO-Zahl.

Die in den Beispielen verwendeten Katalysatoren für die Umwandlung von Epoxiden und Glykolen zu Phenylacetaldehyden sind:

Katalysator A (erfindungsgemäß)

Der Aluminosilikatzeolith vom Pentasil-Typ wurde unter hydrothermalen Bedingungen bei autogenem Druck und 150°C aus 650 g hochdispersem $SiO_2$, 203 g $Al_2(SO_4)_3 \times 18\ H_2O$ in 10 kg einer wäßrigen 1,6-Hexandiamin-Lösung (Mischung 50:50 Gew.%) in einem Rührautoklaven hergestellt. Nach Abfiltrieren und Auswaschen wurde das kristalline Reaktionsprodukt bei 110°C/24 h getrocknet und bei 500°C/24 h calciniert. Dieser Aluminosilikatzeolith enthielt 92,8 Gew.% $SiO_2$ und 4,2 Gew.% $Al_2O_3$.

Katalysator A wird erhalten, indem man den reinen Aluminosilikatzeolithen des Pentasil-Typs mit Verformungshilfsmitteln zu 2-mm-Strängen verformt, bei 110°C/16 h trocknet und bei 500°C/24 h calciniert.

Katalysator B (erfindungsgemäß)

Der Borosilikatzeolith des Pentasil-Typs wird in einer hydrothermalen Synthese aus 640 g hochdispersem $SiO_2$, 122 g $H_3BO_3$, 8 kg einer wäßrigen 1,6-Hexandiamin-Lösung (Mischung 50:50 Gew.%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit Verformungshilfsmitteln 2 mm-Stränge hergestellt, die bei 110°C/16 h getrocknet und bei 500°C/24 h calciniert werden.

Katalysator C (erfindungsgemäß)

Katalysator C wird hergestellt, indem man Katalysator B mit $Cs_2CO_3$ dotiert, bei 130°C/2 h trocknet und bei 540°C/2 h calciniert. Der Cs-Gehalt beträgt 1,3 Gew.%.

Katalysator D (erfindungsgemäß)

Katalysator D wird aus dem kommerziell erhältlichen Mordenit (Zeolon 900 H®) hergestellt, wobei ein Ionenaustausch mit 20 %iger Ammoniumchlorid vorgenommen wird, um den Restnatriumgehalt auf 0,025 Gew.% und weniger zu drücken (Na-Wert nach Trocknung bei 110°C und Calcination bei 500°C).

Katalysator E (erfindungsgemäß)

Bei der Herstellung. des Katalysators E wird kommerziell erhältliches Gemisch Erionit/Chabazit (Zeolon 500®) mit 20 %iger Ammoniumchloridlösung solange ionenausgetauscht, bis das bei 500°C calcinierte Material einen Restnatriumgehalt von 0,11 Gew.% oder weniger aufweist.

Katalysator F (erfindungsgemäß)

Kommerziell erhältlicher L-Zeolith (Baylith L®) wird mit Boehmit im Gewichtsverhältnis 80:20 zu 2 mm-Strängen verformt. Nach Trocknen bei 110°C/16 h und Calcination bei 500°C/16 h liegt der fertige Katalysator F vor.

Katalysator G (Vergleichskatalysator)

Katalysator G wird erhalten, indem man kommerziell erhältlichen Chinopthilolith (Zeolon 400®) mit 20 %iger Ammoniumchloridlösung solange ionenaustauscht bis das bei 500°C calcinierte Produkt einen Restnatriumgehalt von 0,13 Gew.% oder weniger aufweist.

Katalysator H (Vergleichskatalysator)

Kommerziell erhältlicher NaY-Zeolith wird Boehmit im Gewichtsverhältnis 60:40 verstrangt, bei 110°C getrocknet und bei 500°C/16 h calciniert und mit 20 %iger Ammoniumchloridlösung einem Ionenaustausch unterworfen. Der Restnatriumgehalt des Kattalysators H beträgt 0,2 Gew.% (500°C calciniert) oder weniger.

Katalysator I (Vergleichskatalysator)

Boehmit wird mit Verformungshilfsmitteln zu 2 mm-Strängen verformt, bei 110°C/16 h getrocknet und bei 500°C/16 h calciniert.

Katalysator J (Vergleichskatalysator)

Nioboxydhydrat wird mit hochdispersem $SiO_2$ im Gewichtsverhältnis 70:30 zu 2 mm-Strängen verformt. Die Stränge werden bei 110°C getrocknet und bei 300°C/2 h calciniert.

Katalysator K (Vergleichskatalysator)

Nioboxyhydrat wird mit hochdispersem $SiO_2$ im Gewichtsverhältnis 80:20 zu 2 mm-Strängen verformt. Die Stränge werden bei 110°C getrocknet und bei 500°C/16 h calciniert.

Katalysator L (Vergleichskatalysator)

Katalysator L ist ein gefälltes Aluminiumphosphat, das durch Fällung aus $Al(NO_3)_3$-$H_3PO_4$-Lösung mit $NH_3$ bei pH = 6-7 erhalten wird. Nach Abfiltrieren des Niederschlags wird bei 110°C getrocknet und bei 500°C calciniert. Katalysator L enthält 28,5 Gew.% Al und 13,2 Gew.% P.

Katalysator M (erfindungsgemäß)

$AlPO_4$-9 (APO-9) wird synthetisiert, indem man 200 g 98 %ige Phosphorsäure und 136 g Boehmit in 400 g Wasser löst bzw. suspendiert, hierzu eine wäßrige Lösung aus 112 g Diazabicyclo-2,2,2-octan (DABCO) und 320 g $H_2O$ zugibt und diese Mischung in einem Rührautoklaven bei 200°C während 336 Std. unter autogenem Druck umsetzt. Nach Abfiltrieren wird das kristalline Material bei 120°C getrocknet und 500°C/16 h calciniert. Das so synthetisierte $AlPO_4$-9 enthält 49,0 Gew.%$P_2$O5, 37,1 Gew.% $Al_2O_3$. Dieses Material wird mit Verstrangungshilfsmittel zu 3 mm Strängen verformt, abermals bei 120°C getrocknet und bei 500°C/6 h calciniert.

Katalysator N (erfindungsgemäß)

Bei der Synthese von $AlPO_4$-21 (APO-21) erfolgt durch Zusammenrühren von 200 g 98 %ige Phosphorsäure, 156 g gefälltes Aluminiumhydroxid und 71 g Pyrrolidon in 900 g Wasser und anschließende Reaktion bei 200°C unter autogenem Druck während 91 Std. Das bei 120°C getrocknete und bei 500°C calcinierte Produkt enthält 56,6 Gew.% $P_2O_5$ und 43,4 Gew.% $Al_2O_3$. Dieses $AlPO_4$-21 wird mit Verstrangungshilfsmitteln zu 2 mm-Strängen verformt, bei 110°C getrocknet und bei 500°C/16 h calciniert.

Katalysator O (erfindungsgemäß)

$AlPO_4$-5 (SAPO-5) wird hergestellt aus einer Mischung aus 200 g 98 %ige Phosphorsäure, 136 Boehmit, 60 g Kieselsol (30 %ig) 287 g Tripropylamin und 587 g $H_2O$. Diese Mischung wird bei 150°C während 168 Stunden unter autogenem Druck umgesetzt. Nach Filtration wird das kristalline Produkt bei 120°C getrocknet und bei 500°C calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfsmittel zu 3 mm Strängen verformt, bei 120°C getrocknet und bei 500°C calciniert.

Katalysator P (erfindungsgemäß)

$AlPO_4$-11 (SAPO-11) wird synthetisiert aus einer Mischung von 200 g $H_3PO_4$, 136 g AlOOH, 60 g Kieselsol (30 %ig), 91 g Dipropylamin und 890 g Wasser. Die Umsetzung wird bei 200°C während 96 Std. unter autogenem Druck durchgeführt. Nach Filtrieren wird bei 120°C getrocknet und bei 500°C calciniert. SAPO-11 enthäl 457,7 Gew.% $P_2O_5$, 39,4 Gew.% $Al_2O_3$ und 6,4 Gew.% $SiO_2$. Dieses kristalline Produkt wird mit einem Verstrangungshilfsmittel zu 3 mm Strängen verformt bei 120°C getrocknet und bei 500°C/16 h calciniert.

Katalysator Q (erfindungsgemäß)

$BPO_4$ wird hergestellt, indem man 49 g $H_3BO_4$ mit 117 g $H_3PO_4$ (75 %ig) in einem Kneter zusammengibt, überschüssiges Wasser abdampft und das Umsetzungsprodukt zu 3 mm Strängen verformt. Diese Stränge werden bei 100°C getrocknet und bei 350°C calciniert. Katalysator Q enthält 8,77 Gew.% B und 28,3 Gew.% P.

Katalysator R (erfindungsgemäß)

$CePO_4$ wird durch Fällung aus 52 g $Ce(NO_3)_3 \times 6H_2O$ und 56 g $NaH_2PO_4 \times 2H_2O$ erhalten. Nach der Filtration wird das Material zu Strängen verformt, bei 120°C getrocknet und bei 450°C calciniert. Katalysator R enthält 47,1 Gew.% Ce und 12,7 Gew.% P.

Katalysator S (erfindungsgemäß)

Bims wird mit $H_3PO_4$ (75 %ig) getränkt, bei 120°C getrocknet und bei 450°C calciniert. Der $H_3PO_4$-Gehal des Katalysators S beträgt 5 Gew.%.

Katalysator T (Vergleichskatalysator)

Tonsil AC® (Bentonit)

Katalysator U (erfindungsgemäß)

$SiO_2$ (D11-11®) wird mit $NaH_2PO_4 \times 2H_2O$ getränkt, bei 120°C getrocknet und bei 400°C calciniert.

Katalysator V (erfindungsgemäß)

D 11-11® wird mit $H_3BO_3$ gelöst $CH_3OH$ getränkt, bei 120°C getrocknet und 500°C/15 h calciniert. Der Borgehalt beträgt 3,0 Gew.% ($B_2O_3$).

Katalysator W (erfindungsgemäß)

KC-Trockenperlen WS® mit ca. 97 % $SiO_2$ und ca. 3 % $Al_2O_3$ werden mit $H_3BO_3$ gelöst und $CH_3OH$ getränkt, bei 110°C getrocknet und bei 500°C/5 h calciniert. Der Borgehalt beträgt 15,7 Gew.% ($B_2O_3$).

Katalysator X (Vergleichskatalysator)

$TiO_2$ P25® wird mit Verstrangungshilfsmitteln zu 2 mm Strängen verformt und bei 110°C getrocknet und bei 500°C/16 h calciniert.

Katalysator Y (Vergleichskatalysator)

ZnO R5-10®
Die mit diesen Katalysatoren erzielten Versuchsergebnisse sind in Tabelle 1 bis 6 zusammengestellt.

Beispiele 1 bis 6

In den Beispielen 1 bis 5 (Tabelle 1) wird die Temperaturabhängigkeit der Reaktion Styroloxid zu Phenylacetaldehyd veranschaulicht. Beispiel 6 (Tabelle 1) ist ein Vergleichsbeispiel. Hierbei wird auch ersichtlich, daß die erfindungsgemäßen Zeolithe die Bildung des trimeren Phenylacetaldehyd unterdrücken.

Beispiel 7 bis 15

In den Beispielen 7 bis 13 (Tabelle 2) wird die Isomerisierung von Styroloxid zu Phenylacetaldehyd an verschiedenen erfindungsgemäßen zeolithischen Katalysatoren dargestellt. In den Beispielen 14 bis 15 (Tabelle 2) wird die gleiche Reaktion an zeolithischen Vergleichskatalysatoren durchgeführt.
Hieraus geht hervor, daß nicht jeder zeolithische Katalysator für das erfindungsgemäße Verfahren gleich gut geeignet ist.

Beispiele 16 bis 24

In den Beispielen 16 bis 23 (Tabelle 3) wird die Umlagerung an nicht-zeolithischen Katalysatoren auf Basis Nioboxid, gefällten und hydrothermal hergestellten Aluminiumphosphate, Siliciumaluminiumphosphate und Borphosphate. Die Beispiele 18 und 24 zeigen, daß nicht jedes Phosphat (Tabelle 3) und die Beispiele 16 bis 17, daß nicht jedes Metalloxid (Tabelle 3) gleich gut für die Umlagerung geeignet ist.

Beispiele 25 bis 30

Beispiele 25 bis 30 zeigen die Umlagerung von Styroloxid zu Phenylacetaldehyd an mit $H_3PO_4$ bzw. $H_3BO_3$ getränkten Trägermaterialien und an reinen Trägern (Tabelle 4). Nicht jedes saure Oxid ist für Umlagerung geeignet (vgl. auch Tabelle mit Katalysator).

Beispiele 31 bis 40

Die Beispiele 31 bis 40 (Tabelle 5) zeigen die Versuchsergebnisse der Umsetzung von am Aromatenkern substituierten Styroloxide zu den entsprechenden Phenylacetaldehyden.

Beispiel 41

In Beispiel 41 wird ein Standzeitversuch mit Katalysator A bzw. B beschrieben. Ein Quarzglasofen mit 200 ml Füllvolumen und 25 mm Innendurchmesser wird mit Katalysator A oder B gefüllt. Dieser Standzeittest zeigt zugleich auch die Flexibilität der Katalysatoren A und B hinsichtlich verschiedener Einsatzstoffe, die ohne Zwischenregenerierung des Katalysators nacheinander mit hoher Ausbeute umgesetzt wurden. Die Ergebnisse sind in Tabelle 6a und 6b aufgeführt.

Beispiele 42 bis 51

In diesen Beispielen wird die Umsetzung von Phenylglykol, Phenylglykolmonomethylether, Phenylglykolmonophenylether bzw. Phenylglykolmonoessigsäureester zu Phenylacetaldehyd an den Katalysatoren A und B (Tabelle 7) dargestellt.

Beispiel 52

In Beispiel 52 wird die Herstellung von 4-Chlorphenyl-acetaldehyd beschrieben.

Eine Mischung bestehend aus 90 Teilen 4-Chlorphenyl-acetylchlorid, 360 Teilen Xylol und 4 Teilen eines mit Chinolin/Schwefel vergifteten Palladium-Katalysators (10 % Pd auf Kohle) wird bei 100°C 1,5 Stunden mit 25 l/g Wasserstoff begast.

Das Abgas wird in Wasser eingeleitet und der dabei in Lösung gegangene Chlorwasserstoff mit Natronlauge neutralisiert. Aus dem Natronlaugeverbrauch errechnet sich ein Umsatz von 98,4%.

Mittels CO-Zahl und GC wurde im filtrierten Reaktionsgemisch (430 Teile) ein Gehalt von 15,4% 4-Chlorphenyl-acetaldehyd bestimmt. Das entspricht einer Ausbeute von 92 % der Theorie.

Beispiel 53

Hier wird die Herstellung von 2-Chlor-6-fluor-phenyl-acetaldehyd beschrieben.

Arbeitet man wie im Beispiel 52 beschrieben, verwendet jedoch 90 Teile 2-Chlor-6-fluor-phenyl-acetylchlorid, so erhält man 435 Teile Reaktionslösung, die 14,8% 2-Chlor-6-fluor-phenyl-acetaldehyd (ermittelt durch CO-Zahl und Gaschromatogramm) enthält. Die Ausbeute beträgt somit 86 % der Theorie. Der Umsatz, bestimmt aus dem Natronlaugeverbrauch des Abgases, liegt bei 98,3 %.

Beispiel 54

Die Herstellung von 3-(Trifluormethyl-phenyl)-acetaldehyd nach Rosenmund-Reduktion ist im Beispiel 54 gezeigt.

Arbeitet man wie im Beispiel 52 beschrieben, verwendet jedoch 90 Teile 3-(Trifluormethyl-phenyl)-acetylchlorid, so erhält man 432 Teile Reaktionslösung, die 15,6 % 3-(Trifluormethyl-phenyl)acetaldehyd (ermittelt durch CO-Zahl und Gaschromatogramm) enthält. Somit beträgt die Ausbeute 89 % der Theorie. Der Umsatz, bestimmt aus dem Natronlaugeverbrauch des Abgases, liegt 99 %.

Tabelle 1: Styroloxid zu Phenylacetaldehyd

| Beispiel | 1 | 2 | 3 | 4 | 5 | 6*) |
|---|---|---|---|---|---|---|
| Katalysator | A | A | A | A | A | I |
| Temperatur | 200°C | 250°C | 300°C | 350°C | 400°C | 300°C |
| WHSV | 3,0 $h^{-1}$ | 3,0 $h^{-1}$ | 3,1 $h^{-1}$ | 3,4 $h^{-1}$ | 3,2 $h^{-1}$ | 2,7 $h^{-1}$ |
| Umsatz % | 100 | 100 | 100 | 100 | 100 | 100 |
| Selektivität | | | | | | |
| Prod. 1[1)] | 90,1 | 94,5 | 98,0 | 94,8 | 92,3 | 68,9 |
| Prod. 2[2)] | 7,6 | 4,3 | 1,5 | 3,4 | 3,1 | 17,0 |
| Laufzeit | 6 h | 6 h | 102 h[3)] | 54 h[3)] | 6 h | 6 h |

*) Vergleichsbeispiel
1) Phenylacetaldehyd
2) trimeres Phenylacetaldehyd
3) keine Desaktivierung feststellbar

Tabelle 2: Styroloxid zu Phenylacetaldehyd (I) an zeolithischen Katalysatoren

| Beispiel | 8 | 9 | 10 | 11 | 12 | 13 | 14*) | 15*) |
|---|---|---|---|---|---|---|---|---|
| Katalysator | A | B | C | D | E | F | G | H |
| Temperatur | 300°C | 300°C | 300°C | 300°C | 300°C | 300°C | 300°C | 300°C |
| WHSV | 3,1 $h^{-1}$ | 3,0 $h^{-1}$ | 2,1 $h^{-1}$ | 2,2 $h^{-1}$ | 2,2 $h^{-1}$ | 2,2 $h^{-1}$ | 2,2 $h^{-1}$ | 2,3 $h^{-1}$ |
| Umsatz % | 100 | 100 | 100 | 100 | 100 | 100 | 100 | 100 |
| Selektivität % | | | | | | | | |
| I | 99,5 | 96,9 | 98,0 | 92,8 | 93,6 | 91,4 | 85,8 | 81,3 |
| Laufzeit | 6 h | 6 h | 6 h | 6 h | 6 h | 6 h | 6 h | 6 h |

*) Vergleichsbeispiele

Tabelle 3: Styroloxid zu Phenylacetaldehyd (I) an nicht-zeolithischen Katalysatoren

| Beispiel | 16*) | 17*) | 18*) | 19 | 20 | 21 | 22 | 23 | 24*) |
|---|---|---|---|---|---|---|---|---|---|
| Katalysator | J | K | L | M | N | O | P | Q | R |
| Temperatur | 300°C | 300°C | 300°C | 300°C | 300°C | 300°C | 300°C | 300°C | 300°C |
| WHSV | 2,7 $h^{-1}$ | 2,5 $h^{-1}$ | 2,2 $h^{-1}$ | 2,4 $h^{-1}$ | 1,8 $h^{-1}$ | 2,4 $h^{-1}$ | 2,4 $h^{-1}$ | 2,2 $h^{-1}$ | 2,2 $h^{-1}$ |
| Umsatz % | 100 | 100 | 100 | 100 | 100 | 100 | 100 | 100 | 100 |
| Selektivität % | | | | | | | | | |
| I | 82,5 | 82,0 | 84,1 | 92,7 | 89,5 | 92,5 | 89,7 | 97,9 | 79,8 |
| Laufzeit | 6 h | 6 h | 6 h | 6 h | 6 h | 6 h | 6 h | 6 h | 6 h |

*) Vergleichsbeispiele

Tabelle 4: Umlagerung von Styroloxid zu Phenylacetaldehyd an nicht-zeolithischen Katalysatoren

| Beispiel | 25*) | 26 | 27 | 28 | 29*) | 30*) |
|---|---|---|---|---|---|---|
| Katalysator | T | U | V | W | X | Y |
| Temperatur | 300°C | 300°C | 300°C | 300°C | 300°C | 300°C |
| WHSV | 2,1 $h^{-1}$ | 2,4 $h^{-1}$ | 2,4 h–1 | 2,4 $h^{-1}$ | 2,2 $h^{-1}$ | 2,2 $h^{-1}$ |
| Umsatz % | 91,2 | 100 | 100 | 100 | 98,0 | 83,1 |
| Selektivität % | | | | | | |
| I | 89,3 | 94,5 | 95,4 | 96,4 | 74,2 | 79,1 |
| Laufzeit | 6 h | 6 h | 6 h | 6 h | 6 h | 6 h |

*) Vergleichsbeispiel

Tabelle 5

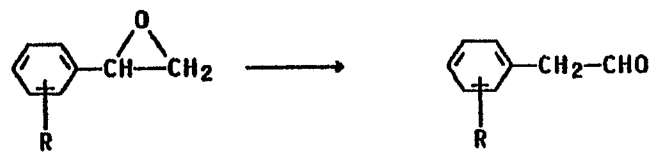

(I)

| Beispiel | 31 | 32 | 33 | 34 | 35 | 36 | 37 | 38 | 39 | 40 |
|---|---|---|---|---|---|---|---|---|---|---|
| Katalysator | A | A | A | A | B | A | A | A | B | Q |
| R | 4-Fluor | 2,4-Di-fluor | 3,4-Di-fluor | 2,4-Di-chlor | 2,4-Di-chlor 2) | 3,4-Di-chlor 1) | 4-Tri-fluor-methyl 1) | 2-Me-thyl 2) | 2-Me-thyl 2) | 2-Me-thyl 2) |
| Temperatur | 300°C | 300°C | 250°C | 300°C | 300°C | 300°C | 300°C | 300°C | 300°C | 300°C |
| WHSV | 2,8 $h^{-1}$ | 2,9 $h^{-1}$ | 2,5 $h^{-1}$ | 3,1 $h^{-1}$ | 3,0 $h^{-1}$ | 3,0 $h^{-1}$ | 3,0 $h^{-1}$ | 2,5 $h^{-1}$ | 2,5 $h^{-1}$ | 2,5 $h^{-1}$ |
| Umsatz % | 100 | 100 | 100 | 100 | 100 | 100 | 100 | 100 | 100 | 100 |
| Selektivität % | | | | | | | | | | |
| I | 93,6 | 85,2 | 82,0 | 84,9 | 89,0 | 91,5 | 94,0 | 92,5 | 92,9 | 92,1 |
| Laufzeit | 6 h | 6 h | 6 h | 6 h | 6 h | 6 h | 6 h | 6 h | 6 h | 6 h |

1) gelöst in Toluol; 50 %ige Lösung
2) gelöst in THF; 50 %ige Lösung

Tabelle 6a: Beispiel 41: Standzeitversuch am Aluminosilikatzeolith des Pentasiltyps (Katalysator A) mit wechselnden Einsatzstoffen, keine Zwischenregenerierung

| Einsatzstoffe | Styrol-oxid | – | – | – | – | 4-Fluor-styrol-oxid | – | Verb. II[3] | Verb. III3) | Styrol-oxid |
|---|---|---|---|---|---|---|---|---|---|---|
| Gesamtlaufzeit | 17 h | 32 h | 52 h | 62 h | 77 h | 87 h | 97 h | 110 h | 115 h | 122 h |
| Laufzeit | 17 h | 32 h | 52 h | 62 h | 77 h | 10 h | 20 h | 3 h | 3 h | 5 h |
| Temperatur | 300°C | 300°C | 300°C | 300°C | 300°C | 300°C | 300°C | 300°C | 300°C | 300°C |
| WHSV | 0,2 $h^{-1}$ | 0,2 $h^{-1}$ | 0,2 h–1 | 0,2 $h^{-1}$ | 0,2 $h^{-1}$ | 0,2 $h^{-1}$ | 0,2 $h^{-1}$ | 0,2 $h^{-1}$ | 0,2 $h^{-1}$ | 0,2 $h^{-}$ |
| Umsatz % | 100 | 100 | 100 | 100 | 100 | 100 | 100 | 100 | 100 | 100 |
| Selektivität % | | | | | | | | | | |
| Produkt [1] | 97,1 | 98,3 | 98,5 | 98,8 | 97,9 | 91,8 | 89,6 | 95,2 | 90,7 | 94,5 |
| Produkt[2] | 97,5 | 98,0 | 98,4 | 98,5 | 98,6 | 91,0 | 89,4 | / | / | 93,4 |

1) GC-Auswertung
2) CO-Zahl-Auswertung
3) gelöst in Toluol 50%ige Lösung
Verb. II = 2-Trifluor-methylstyroloxid
Verb. III = 3,4-Dichlorstyroloxid

Tabelle 6b: Beispiel 41b: Standzeitversuch am Barosilikatzeolith des Pentasiltyps Katalysator B mit wechselnden Einsatzstoffen; keine Zwischenregenerierung

| Einsatzstoff | Styrol-oxid | Styrol-oxid | 4-Fluor-styrol-oxid | Styrol-oxid | Verb. IV | Verb. IV | Styrol-oxid | Verb. V | Verb. V |
|---|---|---|---|---|---|---|---|---|---|
| Gesamtlaufzeit | 15 h | 25 h | 35 h | 40 h | 45 h | 50 h | 55 h | 60 h | 65 h |
| Laufzeit | 15 h | 25 h | 10 h | 5 h | 5 h | 5 h | 5 h | 5 h | 5 h |
| Temperatur | 300°C | 300°C | 300°C | 300°C | 300°C | 300°C | 300°C | 300°C | 300°C |
| WHSV | 0,4 $h^{-1}$ | 0,4 $h^{-1}$ | 0,3 h–1 | 0,4 $h^{-1}$ | 0,3 $h^{-1}$ | 0,3 $h^{-1}$ | 0,4 $h^{-1}$ | 0,4 $h^{-1}$ | 0,4 $h^{-1}$ |
| Umsatz % | 100 | 100 | 100 | 100 | 100 | 100 | 100 | 100 | 100 |
| Selektivität | | | | | | | | | |
| Produkt[1] | 97,9 | 98,3 | 94,5 | 98,7 | 93,6 | 93,3 | 99,2 | 97,6 | 96,6 |
| Produkt[2] | 97,6 | 97,9 | 86,3[3] | 98,3 | 94,1 | 93,8 | 98,9 | 96,6 | 95,2 |

Tabelle 6b: Fortsetzung

| Einsatzstoff | Styrol-oxid | Styrol-oxid | Verb. VI | Verb. VI | Styrol-oxid | Verb. VII | Styrol-oxid | Verb. VIII | Styrol-oxid |
|---|---|---|---|---|---|---|---|---|---|
| Gesamtlaufzeit | 70 h | 75 h | 80 h | 90 h | 95 h | 100 h | 120 h | 135 h | 130 h |
| Laufzeit | 5 h | 5 h | 5 h | 10 h | 5 h | 5 h | 20 h | 5 h | 5 h |
| Temperatur | 300°C | 300°C | 300°C | 300°C | 300°C | 300°C | 300°C | 300°C | 300°C |
| WHSV | 0,4 $h^{-1}$ | 0,4 $h^{-1}$ | 0,4 h–1 | 0,4 $h^{-1}$ | 0,4 $h^{-1}$ | 1,0 $h^{-1}$ | 0,4 $h^{-1}$ | 0,4 $h^{-1}$ | 0,4 $h^{-1}$ |
| Umsatz % | 100 | 100 | 100 | 100 | 100 | 100 | 100 | 100 | 100 |
| Selektivität | | | | | | | | | |
| Produkt[1)] | 97,2 | 96,7 | 90,0 | 90,7 | 94,6 | 94,1 | 98,2 | 94,7 | 98,1 |
| Produkt[2)] | 98,2 | 98,4 | 86,8[3)] | 85,7[3)] | 96,6 | – | 98,1 | 95,1 | 98,1 |

[1)] GC-Auswertung
[2)] CO-Zahl-Auswertung
[3)] Der Unterschied im Gehalt an Wertprodukt bestimmt durch GC-Auswertung und CO-Zahl rührt von der Bildung des trimeren Produktes her, das jedoch in das Monomere überführt werden kann.
Verb. IV = o-Methyl-p-Fluorstyroloxid
Verb. V = 2,4-Difluorstyroloxid
Verb. VI = 3,4-Difluorstyroloxid
Verb. VII = p-Trifluormethylstyroloxid
Verb. VIII = p-Trifluormethoxistyroloxid

Tabelle 7: Umsetzung von Phenylglykol (IX), Phenylglykolmonoethylether (X), Phenylglykolmonophenylether (XI) und Phenylglykolmonoessigsäureester (XII) zu Phenylacetaldehyd (I)

| Beispiel | 42 | 43 | 44 | 45 | 46 | 47 | 48 | 49 | 50 | 51 |
|---|---|---|---|---|---|---|---|---|---|---|
| Einsatzstoff | IX[1)] | IX[1)] | IX1) | IX[1)] | IX[1)] | IX[1)] | X | XII | XI | XI |
| Katalysator | A | A | A | B | B | B | A | A | A | B |
| Temperatur | 250°C | 300°C | 350°C | 250°C | 300°C | 350°C | 300°C | 300°C | 300°C | 300°C |
| WHSV | 2,4 $h^{-1}$ | 2 $h^{-1}$ | 2,4 h–1 | 2 $h^{-1}$ | 2 $h^{-1}$ | 2 $h^{-1}$ | 2 $h^{-1}$ | 1 $h^{-1}$ | 2,1 $h^{-1}$ | 3,5 $h^{-1}$ |
| Umsatz % | 100 | 100 | 100 | 100 | 100 | 100 | 100 | 100 | 100 | 100 |
| Selektivität % | | | | | | | | | | |
| I | 92,0 | 96,4 | 87,6 | 93,2 | 94,4 | 92,2 | 95,1 | 94,7 | 91,9 | 96,3 |

1) Phenylglykol gelöst in THF, 25%ige Lösung

In Tabelle 8 sind die physikalischen und spektroskopischen Daten der neuen substituierten Phenylacetaldehyde wiedergegeben:

| | Kochpunkt | H-NMR-Daten gemessen in $CDCl_3$. |
|---|---|---|
| 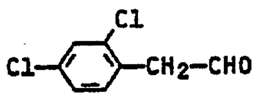 | $80^0C/0,3$ mbar | = 3,82 ppm (2 H, $-CH_2-$)<br>7,0 - 7,33 ppm (2 A, aromat. Protonen)<br>7,45 ppm (1 H, aromat. Proton)<br>9,75 ppm (1 H, -CHO)<br>300 MHz-Gerät |
| 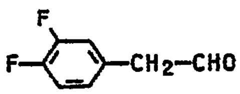 | $91^0C/25$ mbar | = 3,69 ppm (2 H, $-CH_2-$)<br>6,87 - 7,21 ppm (3 A, aromat. Protonen)<br>9,74 ppm (1 H, -CHO)<br>250 MHz-Gerät |
| 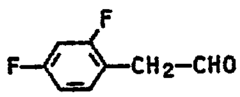 | $93^0C/30$ mbar | = 3,73 ppm (2 H, $-CH_2-$)<br>6,8 - 6,95 ppm (2 A, aromat. Protonen)<br>7,1 - 7,22 ppm (1 H, aromat. Protonen)<br>9,73 ppm (1H, -CHO)<br>250 MHz-Gerät |
| 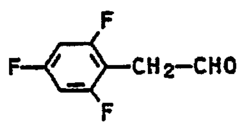 | $81^0C/13$ mbar | |
| 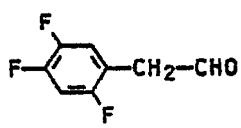 | $56^0C/1$ mbar | |
| 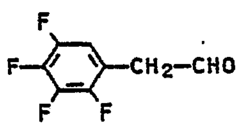 | $87^0C/23$ mbar | |
| 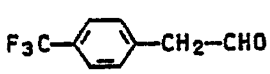 | $109^0C/20$ mbar | = 3,77 ppm (2H)<br>9,77 ppm (1H)<br>250 MHz-Gerät |
| 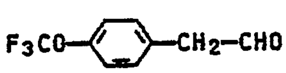 | $77-78^0C/30$ mbar | = 3,71 ppm (2H)<br>7,0 - 7,3 ppm (4H)<br>9,75 ppm (1H)<br>250 MHz-Gerät |
| 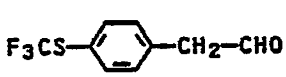 | $88-90^0C/30$ mbar | |
| 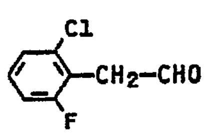 | $102-103^0C/13$ mbar | |

Tabelle 8 Fortsetzung

| | Kochpunkt | H-NMR-Daten gemessen in $CDCl_3$ |
|---|---|---|
| 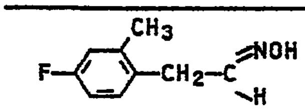 | 1) | = 1,83 und 2,25 ppm (3H)<br>3,47 und 3,67 ppm (2 Dubletts, 1H<br>6,73 und 7,47 ppm (2 Tripletts,1H<br>6,8 und 6,93 ppm (2H)<br>7,05 und 7,17 ppm (1H)<br>8,2 und 8,83 ppm (breit, 1H) |
| 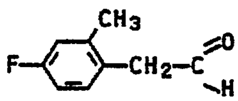 | wurde als Oxim charakterisiert | |

Tabelle 9

Physikalische und spektroskopische Daten von bereits bekannten Phenylacetaldehyden, die nach einem der erfindungsgemäßen Verfahren hergestellt worden sind:

| | Kochpunkt | $^1H$-NMR-Daten gemessen in $CDCl_3$ |
|---|---|---|
| 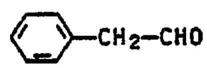 | 193-194°C | |
| 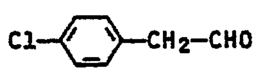 | 100-101°C/18 mbar<br>55°C/0,1 mbar | |
| 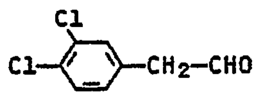 | | = 3,68 ppm (2 H, $-CH_2-$)<br>7,0-7,1 ppm (1 A, aromat. Proton)<br>7,32 ppm (1 H, aromat. Proton)<br>7,4 - 7,5 ppm (1 H, aromat. Proton)<br>9,77 ppm (1 H, -CHO)<br>250 MHz-Gerät |
| 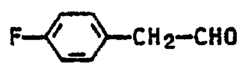 | 37°C/0,25 mbar | = 3,72 ppm (2 H, $-CH_2-$)<br>6,8 - 7,3 ppm (4 H, aromat. Protonen)<br>9,73 ppm (1 H, -CHO, Proton)<br>250 MHz-Gerät |
| 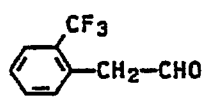 | | = 3,93 ppm (2 H, $-CH_2-$)<br>9,77 ppm (1 H, -CHO)<br>aromatische Protonen lassen sich nicht exakt zuordnen |
| 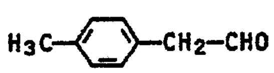 | 106-107°C/18 mbar | |
| 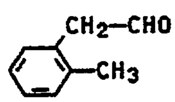 | 95°C/16 mbar | |
| 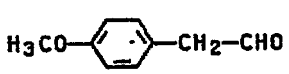 | 116-117°C/13 mbar | |

## Patentansprüche

1. Phenylacetaldehyde der Struktur (I)

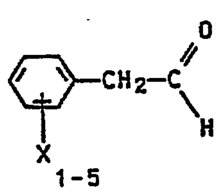

(I)

wobei die Substituenten X Difluor-, Trifluor-, Tetrafluor-, p-Trifluormethyl-, 2-Methyl-4-fluor-, Halogenalkoxy- oder Halogenalkylthio-Reste oder nebeneinander vorhandene Cl, F, $CF_3$, Alkyl-, Alkoxy-, Halogenalkoxy - und/oder Halogenalkylthio-Reste bedeuten.

2. Verfahren zur Herstellung von Phenylacetaldehyden der Struktur (I)

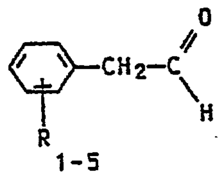

(I)

wobei R Wasserstoff, Alkyl-, Alkoxy-, Halogen-, Halogenalkyl-, Halogenalkoxy- und/oder Halogenalkylthio-Reste bedeuten, dadurch gekennzeichnet, daß man Epoxide der Struktur (II)

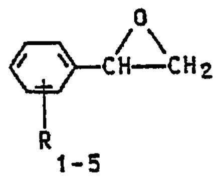

(II)

an Zeolithen des Pentasil-Typs, des Mordenit-Typs oder des Erionit/Chabazit-Typs oder l-Typs katalytisch umlagert.

3. Verfahren nach Anspruch 2, dadurch gekennzeichnet, daß man als Katalysatoren Aluminiumsilikatzeolithe des Pentasiltyps verwendet.

4. Verfahren nach Anspruch 2, dadurch gekennzeichnet, daß man als Katalysatoren Borosilikatzeolithe des Pentasiltyps verwendet.

5. Verfahren zur Herstellung von Phenylacetaldehyden der Struktur (I)

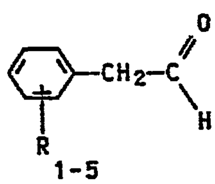

(I)

wobei R Wasserstoff, Alkyl-, Alkoxy-. Halogen-, Halogenalkyl-, Halogenalkoxy- und/oder Halogenalkylthio-Reste bedeuten, dadurch gekennzeichnet, daß man Epoxide der Struktur (II)

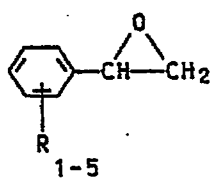

(II)

an Borphosphaten oder an hydrothermal hergestellten Aluminiumphosphaten, Siliciumaluminiumphosphaten oder Eisensiliziumaluminiumphosphaten katalytisch umlagert.

6. Verfahren zur Herstellung von Phenylacetaldehyden der Struktur (I)

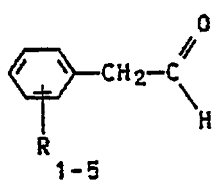

(I)

wobei R Halogen-, Halogenalkyl-, Halogenalkoxy- und/oder Halogenalkylthio-Reste bedeuten, dadurch gekennzeichnet, daß man Epoxide der Struktur (II)

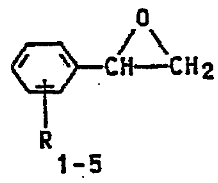

(II)

an Phosphorsäure auf Bims bzw. Siliciumdioxid bzw. Aluminiumoxid oder an Borsäure auf Bims bzw. Siliciumdioxid bzw. Aluminiumoxid katalytisch umlagert.

7. Verfahren zur Herstellung von Phenylacetaldehyden der Struktur (I)

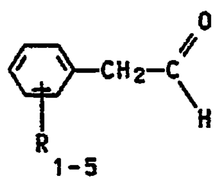

(I)

wobei R Wasserstoff, Alkyl-, Alkoxy-, Halogen-, Halogenalkyl-, Halogenalkoxy- und/oder Halogenalkylthio-Reste bedeuten dadurch gekennzeichnet, daß man Verbindungen der Struktur (III)

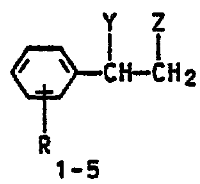

(III)

wobei Y und Z gleich oder verschieden sein können und Hydroxy-, Alkoxy-, Aryloxy- und Acyloxy-Reste bedeuten können, an Zeolithen des Pentasil-Typs, des Mordenit-Typs oder des Erionit-/Chabazid-Typs oder L-Typs katalytisch umlagert.

8. Verfahren zur Herstellung von Phenylacetaldehyden der Struktur (I)

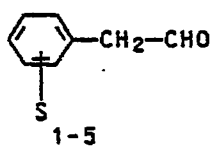

(I)

wobei S Halogen-, Halogenalkyl-, Halogenalkoxy- und/oder Halogenalkylthio-Reste bedeuten, dadurch gekennzeichnet, daß man Phenylessigsäurechloride der Struktur (IV)

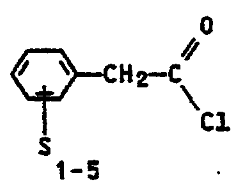

(IV)

in Gegenwart eines mit Chinolin/Schwefel behandelten Palladiumkatalysators in der Flüssigphase hydriert.

**Claims**

1. A phenylacetaldehyde of the structure (I)

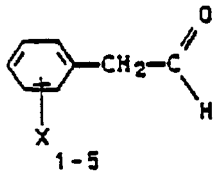

(I)

where the substituents X are difluoro, trifluoro, tetrafluoro, p-trifluoromethyl, 2-methyl-4-fluoro, haloalkoxy or haloalkylthio radicals or are adjacent Cl, F, $CF_3$, alkyl, alkoxy, haloalkoxy and/or haloalkylthio radicals.

2. A process for the preparation of a phenylacetaldehyde of the structure (I)

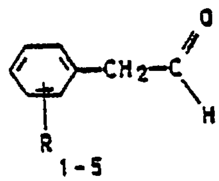

(I)

where R is hydrogen, alkyl, alkoxy, halogen, haloalkyl, haloalkoxy and/or haloalkylthio, wherein an epoxide of the structure (II)

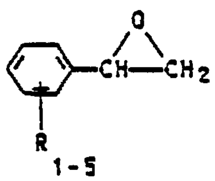

(II)

is subjected to a catalytic rearrangement reaction over a zeolite of the pentasil type, of the mordenite type, of the erionite/chabazite type or of the L type.

3. A process as claimed in claim 2, wherein an aluminosilicate zeolite of the pentasil type is used as the catalyst.

4. A process as claimed in claim 2, wherein a borosilicate zeolite of the pentasil type is used as the catalyst.

5. A process for the preparation of a phenylacetaldehyde of the structure (I)

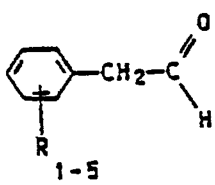

(I)

where R is hydrogen, alkyl, alkoxy, halogen, haloalkyl, haloalkoxy and/or haloalkylthio, wherein an epoxide of the structure (II)

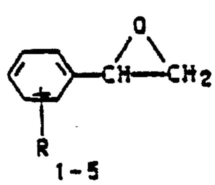

(II)

is subjected to a catalytic rearrangement reaction over a boron phosphate or a hydrothermally prepared aluminum phosphate, silicon aluminum phosphate or iron silicon aluminum phosphate.

6. A process for the preparation of a phenylacetaldehyde of the structure (I)

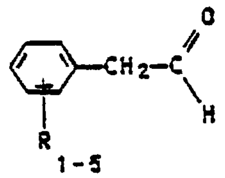

(I)

where R is halogen, haloalkyl, haloalkoxy and/or haloalkylthio, wherein an epoxide of the structure (II)

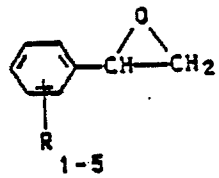

(II)

is subjected to a catalytic rearrangement reaction over phosphoric acid on pumice or silica or alumina, or over boric acid on pumice or silica or alumina.

7. A process for the preparation of a phenylacetaldehyde of the structure (I)

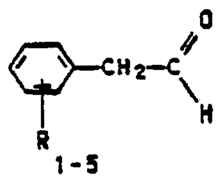

(I)

where R is hydrogen, alkyl, alkoxy, halogen, haloalkyl, haloalkoxy and/or haloalkylthio, wherein a compound of the structure (III)

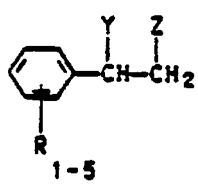

(III)

where Y and Z may be identical or different and are each hydroxyl, alkoxy, aryloxy or acyloxy, is subjected to a catalytic rearrangement reaction over a zeolite of the pentasil type, of the mordenite type, of the erionite/chabazite type or of the L type.

8. A process for the preparation of a phenylacetaldehyde of the structure

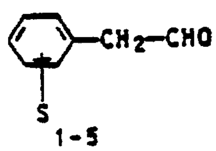

(I)

where S is halogen, haloalkyl, haloalkoxy and/or haloalkylthio, wherein a phenylacetyl chloride of the structure (IV)

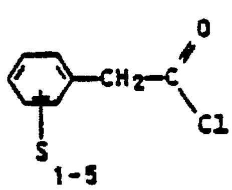

(IV)

is hydrogenated in the liquid phase in the presence of a palladium catalyst treated with quinoline/sulfur.

**Revendications**

1. Phénylacétaldéhydes de structure (I)

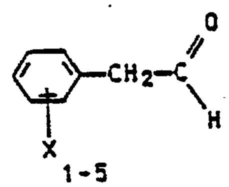

(I.

les substituants X repésentant des restes difluoro, trifluoro, tétrafluoro, p-trifluorométhyle, 2-méthyl-4-fluoro, halogénoalcoxy ou halogénoalkylthio, ou des restes Cl, F, $CF_3$, alkyle, alcoxy, halogénoalcoxy et/ou halogénoalkylthio présents simultanément.

2. Procédé de préparation de phénylacétaldéhydes de structure (I)

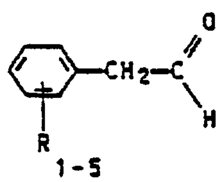

(I)

les substituants R représentant des atomes d'hydrogène, des restes alkyle, alcoxy, halogène, halogénoalkyle, halogénoalcoxy et/ou halogénoalkylthio, caractérisé en ce qu'on réarrange catalytiquement des époxydes de structure (II)

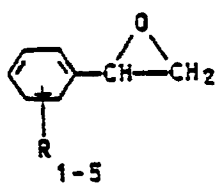

(II)

en présence de zéolithes du type pentasil, du type mordénite, du type érionite/chabazite ou du type L.

3. Procédé selon la revendication 2, caractérisé en ce qu'on utilise, comme catalyseurs, des zéolithes d'aluminosilicate du type pentasil.

4. Procédé selon la revendication 2, caractérisé en ce qu'on utilise, comme catalyseurs, des zéolithes de borosilicate du type pentasil.

5. Procédé de préparation de phénylacétaldéhydes de structure (I)

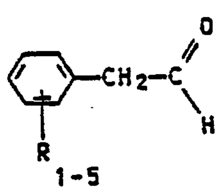

(I)

les substituants R représentant des atomes d'hydrogène, des restes alkyle, alcoxy, halogène, halogénoalkyle, halogénoalcoxy et/ou halogénoalkylthio, caractérisé en ce qu'on réarrange catalytiquement des époxydes de structure (II)

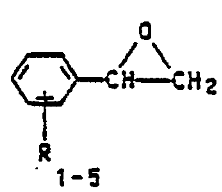

(II)

en présence de phosphates de bore ou en présence de phosphates d'aluminium, de phosphates de silicium-aluminium ou de phosphates de fer-silicium-aluminium préparés par synthèse hydrothermale.

6. Procédé de préparation de phénylacétaldéhydes de structure (I)

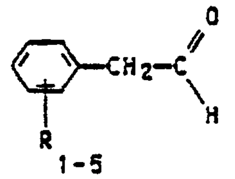 (I)

les substituants R représentant des restes halogène, halogénoalkyle, halogénoalcoxy et/ou halogénoalkylthio, caractérisé en ce qu'on réarrange catalytiquement des époxydes de structure (II)

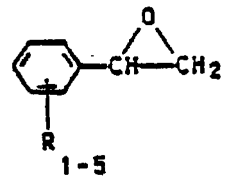 (II)

en présence d'acide phosphorique sur de la pierre ponce, du dioxyde de silicium ou de l'oxyde d'aluminium ou en présence d'acide borique sur de la pierre ponce, du dioxyde de silicium ou de l'oxyde d'aluminium.

7. Procédé de préparation de phénylacétaldéhydes de structure (I)

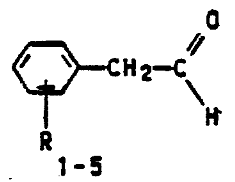 (I)

les substituants R représentant des atomes d'hydrogène, des restes alkyle, alcoxy, halogène, halogénoalkyle, halogénoalcoxy et/ou halogénoalkylthio, caractérisé en ce qu'on réarrange catalytiquement des composés de structure (III)

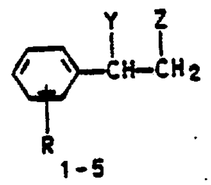 (III)

Y et Z pouvant être identiques ou différents et représentant des restes hydroxy, alcoxy, aryloxy ou acyloxy, en présence de zéolithes du type pentasil, du type mordénite, du type érionite/chabazite ou du type L.

8. Procédé de préparation de phénylacétaldéhydes de structure (I)

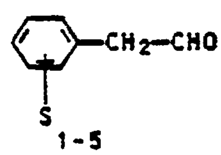 (I)

les substituants S représentant des restes halogène, halogénoalkyle, halogénoalcoxy et/ou halogénoalkylthio, caractérisé en ce qu'on hydrogène en phase liquide des chlorures d'acide phénylacétique de structure (IV)

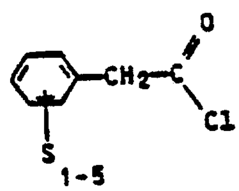 (IV)

en présence d'un catalyseur à base de palladium traité par la quinoléine/soufre.